# EUROPEAN PATENT SPECIFICATION

(11) **EP 2 841 159 B1**
(45) Date of publication and mention of the grant of the patent: **26.07.2017**
(21) Application number: 13781334.1
(22) Date of filing: 14.04.2013
(51) Int. Cl.: A61N 5/06, A61B 18/18, A61C 1/10, H01L 23/552, H05K 9/00, A61C 1/00, A61B 18/22

(54) **AN ELECTROMAGNETIC SHIELD FOR A DENTAL LASER HAND PIECE**
ELEKTROMAGNETISCHE ABSCHIRMUNG FÜR EIN ZAHNÄRZTLICHES LASERHANDWERKZEUG
BLINDAGE ÉLECTROMAGNÉTIQUE POUR UNE PIÈCE À MAIN LASER DENTAIRE

(30) Priority: 24.04.2012 US 201261637377 P
(43) Date of publication of application: 04.03.2015
(73) Proprietor: Light Instruments Ltd., 20692 Yoqneam Illit (IL)
(72) Inventor: WEINBERGER, Yossi, 2199729 Karmiel (IL)
(74) Representative: Sarpi, Maurizio
(86) International application number: PCT/IL2013/000038
(87) International publication number: WO 2013/160888

(56) References cited:
- DE-A1- 4 401 989
- US-A- 5 306 373
- US-A- 6 054 673
- US-A1- 2001 050 175
- US-A1- 2004 259 053
- US-A1- 2007 036 081
- US-A1- 2007 185 553
- US-A1- 2007 265 605
- US-A1- 2008 097 499
- US-A1- 2010 082 076
- US-A1- 2012 021 375
- US-B1- 7 210 947
- US-B2- 6 455 770
- US-B2- 7 842 034
- US-B2- 8 160 707

## Description

### TECHNICAL FIELD

The current method and apparatus relate to electromagnetic shields and in particular to electromagnetic shields for dental laser hand pieces.

### BACKGROUND

Contemporary medical devices involve the wide use of electronic circuits, which generate and emit electromagnetic energy causing unwanted electromagnetic interference (EMI) either by emission or by induction.

The greater the number of devices in the environment and the closer their proximity to each other, as is common in the dental practicing environment, the greater the EMI between the devices. A new source of EMI, especially in the dental environment has been the introduction of laser emitting devices such as dental lasers used for oral surgery and dentistry.

The science of studying Electromagnetic Compatibility (EMC) has been introduced in order to study the effect of EMI generated by the various devices and develop solutions to minimize this effect. Minimizing the EMI is commonly achieved by either reducing the radiation emission from a device at hand; increase the immunity of a device to EMI produced by other, nearby devices or severing the path between two or more devices.

Another element of EMI involves its effect on the safety of operation of dental devices and particularly dental laser devices. EMC has therefore been also aimed towards increasing the safety of the device for device operators as well as for the subjects treated by the devices.

In dental laser devices much of the EMI is generated by laser energy emitters (i.e., a laser pump source paired with a laser medium). In these devices EMC was achieved by accommodating the laser energy emitters inside a base unit and conveying the laser beam to a hand piece via a complex conveying system such as a long optic fiber or an articulated jointed arm including a series of optic fibers and mirrors conveying the laser beam from the laser emitter within the base through a harness to the hand piece from which the laser radiation is emitted. United States Patent Application publication No.2004/0259053 to Bekov et al., discloses a high frequency ultrasonic dental scaler. The scaler includes a laser source situated in a common housing with the fluid pump and the power supply of the scaler apparatus. The laser light is delivered to the treated area through an optical fiber system, which is included into a common hand-piece. The laser light is used to provide a bacteria-free oral environment.

These devices may at times become expensive to manufacture and maintain, as well as, require precise and relatively frequent calibration of the mirrors in the articulated joints. Any attempt to bypass this type of conveying system, such as exteriorizing the laser energy emitter, can bring about in its wake an inevitable increase in EMI.

A document which discloses a dental laser hand piece in which the laser source is included in the hand piece is exemplified by DE4401989A1.

### BRIEF SUMMARY

The invention pertains to a laser energy dental hand piece as defined in claim 1.

Preferred embodiments of the laser hand piece are defined in the dependent claims.

### BRIEF DESCRIPTION OF THE DRAWINGS

The present dental device will be understood and appreciated more fully from the following detailed description, taken in conjunction with the drawings in which:
Fig. 1 is a schematic simplified illustration of a dental laser device in accordance with an example;
Figs. 2A, 2B, 2C, 2D and 2E are sectional view simplified illustrations of a dental laser device hand piece shielding in accordance with an example;
Fig. 3 is a cross-sectional view simplified illustration of a dental laser device harness in accordance with an example;
Figs. 4A and 4B are graphs depicting an effect of a dental hand piece electromagnetic radiation shielding in accordance with an example;
Figs. 5A and 5B are graphs depicting an effect of a dental hand piece electromagnetic radiation shielding in accordance with another example; and
Fig. 6 is a sectional view simplified illustration of a dental laser device hand piece in accordance with an example.

### DETAILED DESCRIPTION

Most of the existing dental laser devices accommodate the laser energy emitters inside a base unit and conveying the laser beam to a hand piece via a complex conveying system such as a long optic fiber or an articulated jointed arm including a series of optic fibers and mirrors conveying the laser beam from the laser emitter within the base through a harness to the hand piece from which the laser radiation is emitted. Such systems are sensitive to temperature, require frequent optical path adjustment, and reduce effective operation time.

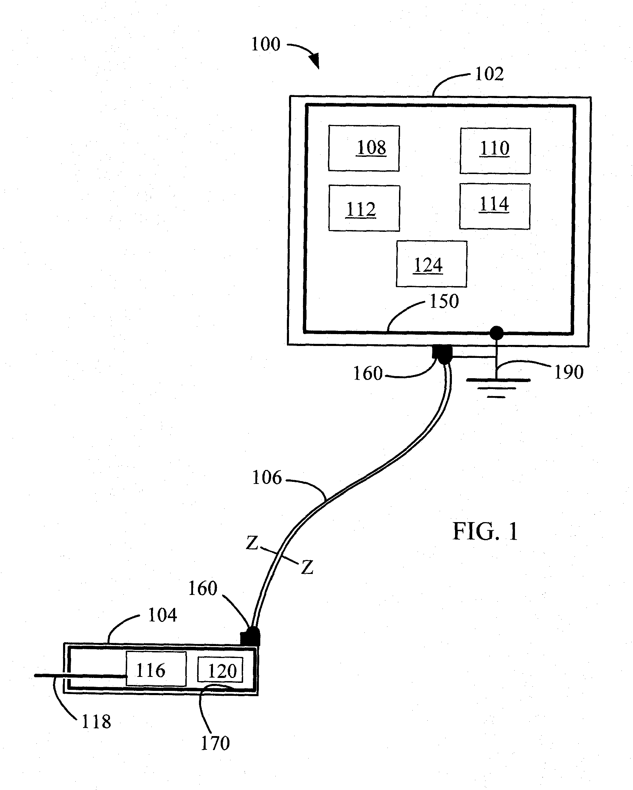

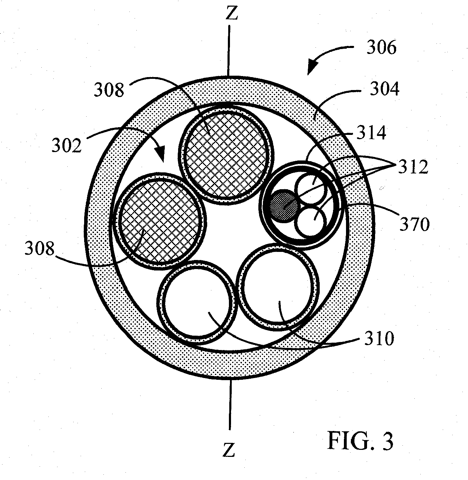

Reference is now made to Fig. 1, which is a schematic simplified illustration of a dental laser device in accordance with an example. A dental device 100 can include a base unit 102 and a hand piece 104 communicating with each other via a harness 106. Harness 106 may be made of flexible tubing, such as an extruded plastic tube, a series of jointed rigid hollow arms or be multi-luminal harness enclosing several separate tubes or conduits within one single lumen (Fig. 3) as will be explained in greater detail below. Base unit 102 can also include a source of power 108, a fluid source reservoir 110, a cooling fluid source 112, a spraying fluid source 124, and a computer 114 governing operation of all of the device parts. Harness 106 in addition to tubes conducting cooling and spraying fluids could include electrical wiring and a conducting layer serving as it will be explained below as a shielding. Both ends of harness 106 could be terminated by couplings 160 which could be known in the art or modified connectors.

Hand piece 104 can include a laser emitter 116 including a solid state laser medium and a laser pump source (not shown) as will be explained in greater detail below and an ID chip 120 supporting recording and calibration of the laser emitter and storing the calibrated values. ID chip 120 communicates with computer 114 and facilitates easy hand piece 104 exchange, identification, and operation. A laser beam generated by laser emitter 116 can be radiated directly from the hand piece or from a sapphire tip 118 in hand piece 104. Additionally, base unit 102, harness 106, couplings or connectors 160, and hand piece 104 could include an electromagnetic interference (EMI) shielding envelope 150 and 170 respectively as will be explained in greater detail below. Harness 106 could also include a similar shielding communicating with the hand piece and base unit.

Location of the laser emitter 116 including a solid state laser medium and a laser pump source in the hand piece simplifies product manufacture, eliminates the need for frequent laser pass folding mirrors adjustment; eliminates frequent optical fiber handling problems casing fiber cracks at extreme operator movements and other related problems. Location of the laser emitter 116 including a solid state laser medium and a laser pump source in the hand piece could however increase the undesired electromagnetic emission produced by the laser dental device.

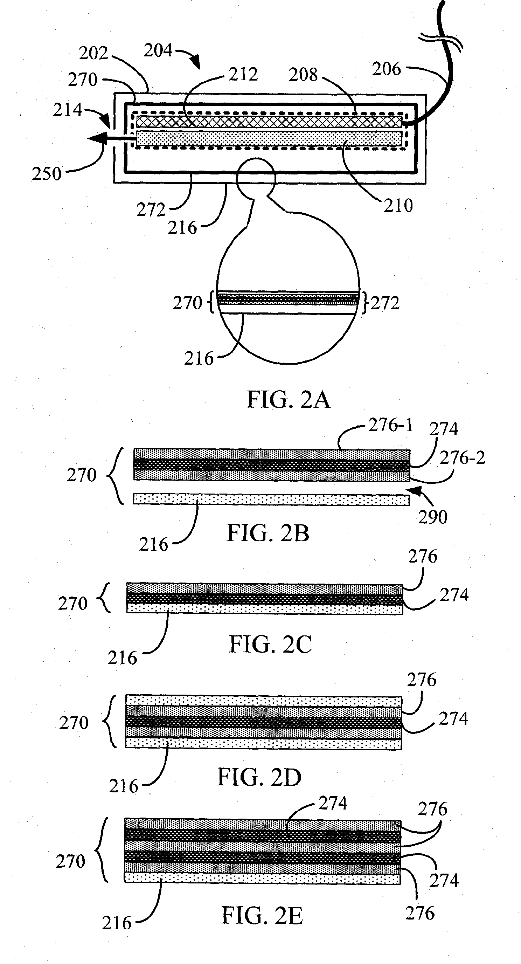

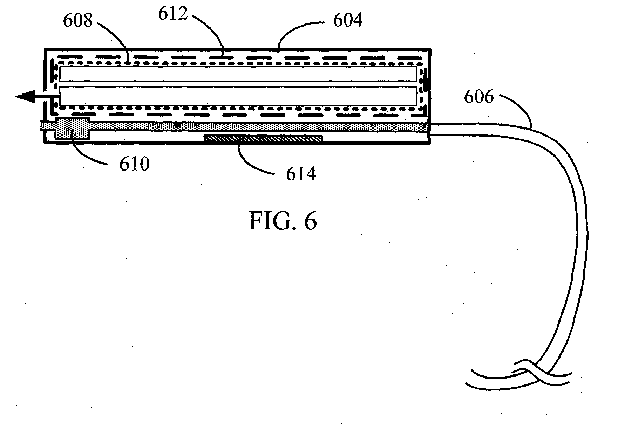

Referring now to Figs. 2A, 2B, 2C and 2D which are sectional view simplified illustrations of a dental laser device hand piece in accordance with an example. As shown in Fig. 2A, dental device hand piece 204, used in a dental device for example, such as that shown in Fig. 1, accommodating a laser emitter 208. Additionally and optionally, hand piece 204 could also include a spraying mechanism or liquid atomizer (Fig. 6), cooling liquid mechanism (Fig. 6) and/or a ID chip 120 (Fig. 1) to identify and facilitate operation of hand piece 204 or provide a user/system operator and computer 114, as well as electronic circuitry with a number of operating parameters.

Laser emitter 208 can include a solid state laser medium or resonator rod 210 paired with a pump source 212 communicating with source of electric power 108 and computer 114 (Fig. 1) via harness 206.

Laser medium 210 commonly used in hand piece 204 is a medium from the Erbium group such as Er:YAG, but laser medium 210 is not necessarily limited to any specific group of laser media and may also employ other laser media commonly used in dentistry such as, Diode laser, Nd:YAG laser and Ho:YAG laser. When excited, laser medium 210 can radiate laser energy (i.e. a laser beam) via an aperture 214 in hand piece 204 housing 202 as indicated by an arrow designated reference numeral 250.

The laser beam emitted by excited laser medium 210 could have a wavelength between 2700 and 3000nm, more commonly between 2800 and 3000nm and most commonly 2940nm. The laser beam could be employed at ablative or sub-ablative power settings. Most commonly, hand piece 204 emitter 208 is employed at a sub-ablative power settings between 0.1 and 100.0W, more commonly between 0.5 and 10.0W and most commonly 8.5W. Additionally, excited laser medium 210 can emit a laser beam at a frequency between 1 and 100Hz, more commonly between 5 and 20Hz and most commonly at 30Hz.

Laser pump source 212 could be a light pump source selected from a group of light pump sources including a flash lamp and an array of laser diodes. Laser pump source 212 flash lamps can be any flash lamp known in the art having a spectrum suitable to excite laser medium 210 such as, for example, a Xenon flash lamp or a Krypton flash lamp. Alternatively and optionally, laser pump source 212 can be any other suitable laser pump source known in the art, such as for example a semiconductor diode array providing a power of 50 to 1000W.

Hand piece 204 also includes a shielding envelope 270 (170, Fig. 1) designed to minimize the effect of EMI generated from electronic circuitry of hand piece 204 by reducing the radiation emission therefrom. Additionally, reducing the radiation emission from hand piece 204 increases the safety of the hand piece and of dental device 100 as a whole for device operators as well as for subjects treated by the device.

Figs. 2B, 2C and 2D are sectional view simplified enlarged illustrations of a section of shielding envelope wall 272 of Fig. 2A. Shielding envelope wall 272 can completely or partially envelope electronic components of hand piece 204 such as laser emitter 208, computer 114 (when applicable), control unit 120 and electronic circuitry thereof (not shown).

As shown in Figs. 2B, 2C and 2D shielding envelope wall 272 can be a multilayered structure including at least one electrical conducting layer 274 and at least one electrical insulating layer 276. The layers may about each other or have a gap 290 in between, the gap can be filled with a medium such as air or fluid or contain a vacuum. United States Patent Application Publication No.2001/0050175 to Pulver discloses methods of manufacture of multilayer EMI shielding layers and their use.

Electrical conducting layer 274 can be made of any electrical conducting metallic material such as copper, nickel, or any other suitable metal in a form of sheet metal, metal screen, metallic ink or metal foam. Electrical conducting layer 274 could be grounded via one or more conductive couplings 160 (Fig. 1) for example, between hand piece 204 and harness 206, or between harness 206 and base 102 (Fig. 1) chassis or shielding 150. Conductive couplings 160 could be of the type known in the art and made in the form of, for example, metal sheet contacts or metal finger-like projections or terminals, such as a combination of Copper-Beryllium finger-like projections In some examples the hand piece could be made of metal and coated by a layer of insulating material, such as epoxy, composite polymer materials, Polyvinyl chloride (PVC) and other materials capable of preventing electric breakdown.

Electrical insulating layer 276 can be made of any insulating material known in the art such as composite polymer materials. Commonly used shielding can be a pliable polyimide film such as Kapton® tape commercially available from DuPont®, Wilmington, Delaware U.S.A. An additional layer of electrical insulating material 278 could also be included in shielding 270, which may also be a pliable polyimide film such as Kapton® tape or any other insulating material such as Polyvinyl chloride (PVC) capable of preventing electric breakdown. In some examples the handpiece could be made of metal and coated by a layer of insulating material, such as epoxy, composite polymer materials, Polyvinyl chloride (PVC) and other materials capable of preventing electric breakdown.

The layers of shielding 270 can be arranged in a layer arrangement, as shown in Fig. 2B, which includes an electrical conducting layer 274 sandwiched between two or more layers of electrical insulating layer 276: an inner electrical insulating layer 276-1 facing hand piece 204 electrical components and an outer layer 276-2 facing or abutting housing 202 wall 216. Housing 202 wall 216 can be made of a rigid or semi-rigid insulating material such as PVC and also serve as part of shielding 270.

As shown in Fig. 2C, shielding 270 could include electrical conducting layer 274 sandwiched between an inner electrical insulating layer 276 and housing 202 wall 216, which in this case also serves as an electrical insulating layer abutting the outer surface of electrical conducting layer 274.

Another combination of shielding 270 layers is shown in Fig. 2D, in which an additional layer of insulation 218 in the form of another insulating material such as, for example, PVC is added to a shielding of the type shown in Fig. 2B.

Fig. 2E depicts a double shielding configuration in which two or more electrical conducting layers 274 are sandwiched between three or more electrical insulating layers 276. In the example of Fig. 2E, metal layers could be deposited on housing 202 wall 216, as well as on connectrors 160 and could add additional insulation and serve as part of general shielding 270.

In configurations combining more than one type of electrical insulating layer, such as a combination of rigid or semi-rigid polymer and pliable polyimide film, the rigid or semi-rigid polymer may also serve as part of shielding 270 and be external to the pliable polyimide film electrical insulating layer.

It will be appreciated by persons skilled in the art that any other layer order configuration of shielding 270 may be applicable providing electrical conducting layer 274 is sandwiched between two or more electrical insulating layers 276 and/or 216.

In a dental laser device, such as device 100 depicted in Fig. 1, having a laser emitter in the hand piece, as shown in Fig. 2, electrical communication (as well as other communication as will be explained in greater detail below) between the hand piece and the base unit is conducted via electrical conductors accommodated within a harness such as harness 106. Such electrical communication may include communication between power source 108 and laser emitter 116, between shielding 170 and device 100 ground 190 as well as between hand piece electronic circuitry (not shown) and computer 114. The electrical conductors being a source of EMI could contribute to the overall emission radiation (EMI) of the dental laser device.

Reference is now made to Fig. 3, which is a cross section of harness 106 such as that depicted in Fig. 1, taken along the axis Z-Z. Harness 106 can be a multi-luminal tube enclosing several lumina made of an extruded polymer or a single lumen tube having a lumen 302 accommodating one or more separate tubes or conduits. Harness 106 wall 304 can be made of any semi-rigid or flexible material such as a polymer (e.g., PVC). Alternatively, harness 306 could be a rigid, articulated, jointed hollow arm. In a dental laser device such as device 100, harness 106 lumen 302 could accommodate additional tubes such as a spray liquid conduit 308 for hand piece 304 spraying mechanism or liquid atomizer (Fig. 6) communication with fluid source reservoir 110 (Fig. 1) and cooling fluid conduits 310 for cooling laser emitter 116 (Fig. 1) communication with cooling fluid source 112 (Fig. 1).

Electrical communication conductors 312 may be accommodated in a tube 314 accommodated within harness 306. A shielding 370, similar in construction to shielding 270 (Figs. 2A, 2B, 2C, 2D and 2E), may envelope conductors 312 and reduce EMI as explained hereinabove.

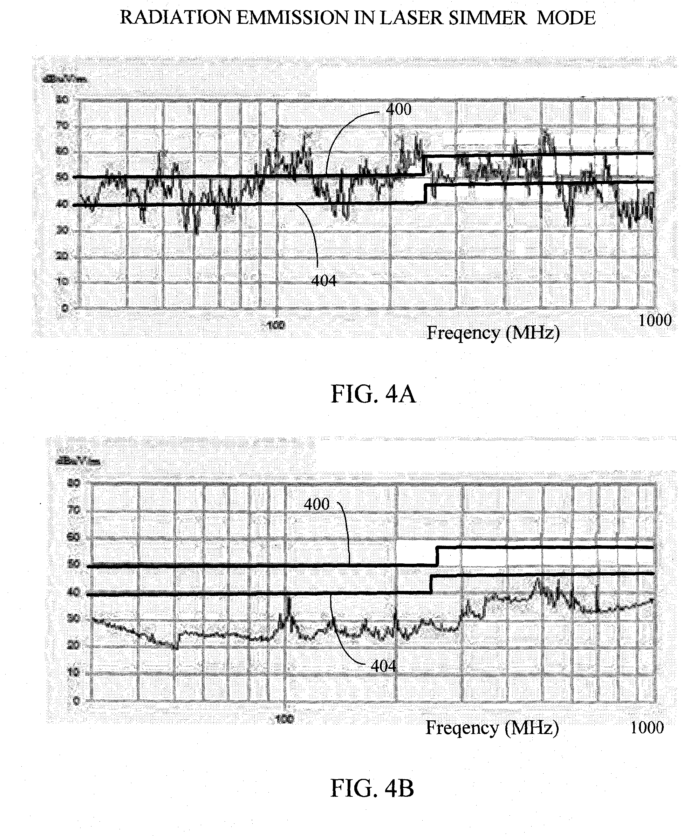

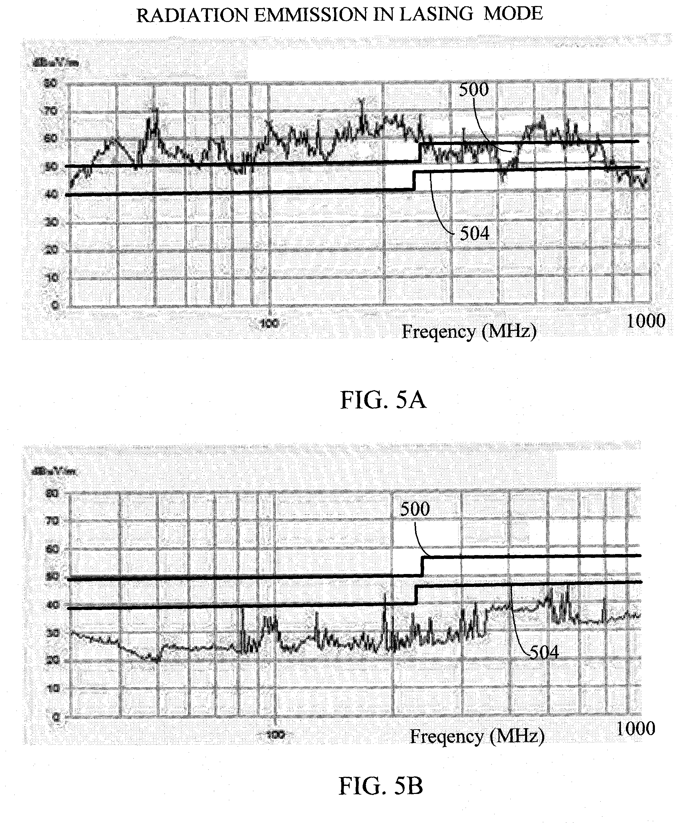

EMI tests measuring radiated emission, performed in accordance with International Standard IEC60601-1-2 related to medical equipment, from a dental laser device having a hand piece such as that described in Figs. 1 and 2, were carried out in a 3 Meter EMC chamber at operating frequencies between 30 and 1000MHz. The test results are shown in graphs provided in Figs. 4A, 4B, 5A and 5B.

Figs. 4A and 4B depict radiation emission (EMI) of the laser emitter of the dental laser device operation circuit in simmer mode operation, which is steady-state operation with partial ionization of the laser pump source (e.g., flash lamp) when the pump source is inactive (e.g., not flashing). The Y-axis depicts the radiation emission (EMI) stated in dB(µV/M) whereas the X-axis depicts the frequency at which the measurements were taken.

Fig. 4A depicts the radiation electromagnetic emission (EMI) of the dental laser device without implementation of shielding 270 (Fig. 2). Line 400 marks the limit for a Class A equipment radiated electromagnetic emission and line 404 marks the limit for a Class B equipment radiated electromagnetic emission. The current laser dental device is class B equipment. A person skilled in the art would appreciate that the emission graph depicts emission fluctuations over the full range of tested frequencies (i.e., 30 to 1000MHz) ranging between 20 and 65 dB(µV/M) in magnitude. The average radiated emission value being at 45 dB(µV/M), and the peak at 100MHz being about 68 dB(µV/M).

The addition of shielding 270 has decreased the radiated emission significantly, as shown in Fig. 4B, to an average radiated emission value being at about 30 dB(µV/M), and the peak at 100MHz being about 40 dB(µV/M). Moreover, significant emission fluctuations have been reduced and as this graph shows cover only a portion of the full range of tested frequencies (i.e., 30 to 1000MHz) as well as dampening the magnitude of the emissions to a magnitude ranging in the most part by a factor between 2 and 10 dB in magnitude (excluding peak fluctuations).

Figs. 5A and 5B depict electromagnetic radiation emission (EMI) of the dental laser device in lasing mode, in which the laser is being excited by the pump source (e.g., flash lamp) to emit laser radiation. At this mode the laser pump source is active (e.g., flashing). The Y-axis depicts the radiation emission (EMI) stated in dB(uV/M) whereas the X-axis depicts the frequency at which the measurements were taken. Line 500 marks the limit for a Class A equipment radiated electromagnetic emission and line 504 marks the limit for a Class B equipment radiated electromagnetic emission. The current laser dental device is class B equipment.

Fig. 5A depicts the radiation emission of the device without implementation of shielding 270 (Fig. 2). As in Fig. 4A, the emission graph in Fig. 5A also depicts emission fluctuations over the full range of tested frequencies (i.e., 30 to 1000MHz) ranging between 50 and 75 dB(µV/M) in magnitude. The average radiated emission value being at about 60 dB(µV/M), and the peak at 100MHz being over 65 dB(µV/M).

The addition of shielding 270 has decreased the radiated emission significantly, as shown in Fig. 5B, to an average radiated emission value being at 25 dB(µV/M), and the peak at 100MHz being about 35 dB(µV/M). Moreover, significant emission fluctuations have been reduced and as this graph shows cover only a portion of the full range of tested frequencies (i.e., 30 to 1000MHz) as well as somewhat dampening the magnitude of the emission fluctuations to a magnitude ranging in the most part between 20 and 30 dB(µV/M) in magnitude (excluding peak fluctuations).

Overall, in the above described test, the average radiated emission (EMI) generated by the dental laser device was reduced by the addition of shielding 270 by a factor of 5 to 10 dB.

In another example of a dental laser device hand piece as shown in Fig. 6, hand piece 604 may also include, in addition to laser emitter 608 a spraying mechanism or liquid atomizer 610.

Liquid atomizer 610 could be used in various endodontic procedures such as, for example, Laser Photomechanical Irrigation technique. Liquid spraying mechanism/atomizer 610 can be supplied via harness 606 spray liquid conduit 308 (Fig. 3) from fluid source reservoir 110 (Fig. 1).

Additionally, hand piece 604 can also include a fluid cooling mechanism 612 for laser emitter 608 communicating with cooling fluid source 112 (Fig. 1) via cooling fluid conduits 310.

Additionally and optionally, hand piece 604 may also include a control unit 614 to control operation of hand piece 604 as well as electronic circuitry.

## Claims

1. A laser energy dental hand piece comprising:
a housing encasing:
at least one laser energy emitter (208), including at least a solid state laser medium (210) and a laser pump source (212); and
an electromagnetic interference (EMI) shield envelope (150, 170, 270).

2. The hand piece (104, 204, 604) according to claim 1, wherein the EMI shield envelope (150, 170, 270) encases at least the laser energy emitter (208).

3. The hand piece (104, 204, 604) according to claim 1, wherein the laser medium (210) is at least one laser medium selected from a group of laser mediums including Erbium lasers.

4. The hand piece (104, 204, 604) according to claim 1, wherein the laser pump source (212) is at least one laser pump source selected from a group of laser pump sources including a flash lamp and an array of laser diodes.

5. The hand piece (104, 204, 604) according to claim 1, wherein the electromagnetic interference shield comprises at least one electrical conducting layer (274) and at least two electrical insulating layers (276-1 and 276-2) and wherein the two electrical insulating layers (276-1 and 276-2) comprise at least one electrical insulating material selected from a group of insulating materials including polyimides, PVC and composite polymers.

6. The hand piece (104, 204, 604) according to claim 1, wherein the electromagnetic interference shield (270) comprises at least one of a layer of copper and a layer of polyimide, both sandwiched by at least two layers of polymer.

7. The hand piece (104, 204, 604) according to claim 6, wherein the electromagnetic interference shield (270) is grounded via at least one electrically conductive coupling (312) coupled at one end to the electrical conducting layer (274) and at the other end to a ground.

8. The hand piece (104, 204, 604) according to claim 1, wherein the hand piece (104, 204, 604) also includes at least one fluid conduit (308, 310) communicating with a source of fluid (124) or a waste deposit reservoir.

9. The hand piece (104, 204, 604) according to claim 1, wherein the hand piece is coupled via a harness (206, 606) to a source of power, a ground, a source of fluid (124) and a waste deposit reservoir and wherein the harness (206, 606) is multi-luminal.

10. The hand piece (104, 204, 604) according to claim 9, wherein the hand piece is conductively coupled to the ground and to the source of power (108) via electrical conductors accommodated in the harness and are also encased in an electromagnetic interference shield.

11. The hand piece (104, 204, 604) according to claim 1, wherein the electromagnetic interference shield (270) reduces the average radiated electromagnetic emission during lasing mode of operation below 18 dB(µV/M).

12. The hand piece (104, 204, 604) according to claim 1, wherein the electromagnetic interference shield (270) reduces the average radiated emission (EMI) generated by the laser dental equipment during lasing mode of operation by a factor of 5 to 10 dB at a frequency of 100MHz.

13. The hand piece (104, 204, 604) according to claim 1, wherein the electromagnetic interference shield (270) reduces the average radiated emission (EMI) generated by the dental laser device during simmer mode of operation below 18 dB(µV/M).

14. The hand piece (104, 204, 604) according to claim 1, wherein the electromagnetic interference shield (270) reduces the average radiated emission (EMI) generated by the dental laser device during simmer mode of operation by a factor of 5 to 10 times as compared to a non-shielded mode of operation at a frequency of 100MHz.

15. The hand piece (104, 204, 604) according to claim 1, wherein the electromagnetic interference shield reduces the magnitude of the EMI fluctuation over at least a portion of operating frequencies between 30 and 1000MHz.

## Patentansprüche

1. Laserenergie-Dentalhandstück, umfassend:
ein Gehäuse, das:
mindestens einen Laserenergiestrahler (208) einschließlich mindestens eines Festkörper-Lasermediums (210) und einer Laserpumpquelle (212); und
eine (EMI)-Schutzumhüllung gegen elektromagnetische Störungen (150, 170, 270) kapselt.

2. Handstück (104, 204, 604) nach Anspruch 1, wobei die EMI-Schutzumhüllung (150, 170, 270) zumindest den Laserenergiestrahler (208) kapselt.

3. Handstück (104, 204, 604) nach Anspruch 1, wobei das Lasermedium (210) mindestens ein Lasermedium ist, das aus einer Gruppe von Lasermedien ausgewählt ist, die Erbium-Laser einschließen.

4. Handstück (104, 204, 604) nach Anspruch 1, wobei die Laserpumpquelle (212) mindestens eine Laserpumpquelle ist, die aus einer Gruppe von Laserpumpquellen ausgewählt ist, die eine Blitzlampe und eine Anordnung von Laserdioden einschließen.

5. Handstück (104, 204, 604) nach Anspruch 1, wobei der Schirm gegen elektromagnetische Störungen mindestens eine elektrische leitende Schicht (274) und mindestens zwei elektrische isolierende Schichten (276-1 und 276-2) aufweist, und wobei die zwei elektrisch isolierenden Schichten (276-1 und 276-2) mindestens einen elektrischen isolierenden Werkstoff aufweisen, der aus einer Gruppe von Isolierwerkstoffen ausgewählt ist, die Polyimide, PVC und Kompositpolymere einschließen.

6. Handstück (104, 204, 604) nach Anspruch 1, wobei der Schirm gegen elektromagnetische Störungen (270) eine Kupferschicht und/oder eine Polyimidschicht aufweist, die beide durch mindestens zwei Polymerschichten schichtenweise angeordnet sind.

7. Handstück (104, 204, 604) nach Anspruch 6, wobei der Schirm gegen elektromagnetische Störungen (270) durch mindestens eine elektrisch leitfähige Kopplung (312) geerdet ist, die an einem Ende mit der elektrischen leitenden Schicht (274) und an dem anderen Ende mit einer Masse gekoppelt ist.

8. Handstück (104, 204, 604) nach Anspruch 1, wobei das Handstück (104, 204, 604) außerdem mindestens eine Fluidleitung (308, 310) einschließt, die mit einer Fluidquelle (124) oder einem Entsorgungsbehälter in Verbindung steht.

9. Handstück (104, 204, 604) nach Anspruch 1, wobei das Handstück über einen Kabelbaum (206, 606) mit einer Stromquelle, einer Masse, einer Fluidquelle (124) und einem Entsorgungsbehälter gekoppelt ist, und wobei der Kabelbaum (206, 606) multi-luminal ist.

10. Handstück (104, 204, 604) nach Anspruch 9, wobei das Handstück leitfähig mit der Masse und mit der Stromquelle (108) über elektrische Leiter gekoppelt ist, die in dem Kabelbaum aufgenommen sind und ebenfalls in einem Schirm gegen elektromagnetische Störungen gekapselt sind.

11. Handstück (104, 204, 604) nach Anspruch 1, wobei der Schirm gegen elektromagnetische Störungen (270) die mittlere abgestrahlte elektromagnetische Emission während des Laserbetriebsmodus unter 18 dB (µV/M) reduziert.

12. Handstück (104, 204, 604) nach Anspruch 1, wobei der Schirm gegen elektromagnetische Störungen (270) die mittlere abgestrahlte Emission (EMI), die durch die zahnärztliche Laserausrüstung während des Laserbetriebsmodus erzeugt wird, um einen Faktor von 5 bis 10 dB bei einer Frequenz von 100 MHz reduziert.

13. Handstück (104, 204, 604) nach Anspruch 1, wobei der Schirm gegen elektromagnetische Störungen (270) die mittlere abgestrahlte Emission (EMI), die durch das zahnärztliche Lasergerät während des Leichtsiedebetriebsmodus erzeugt wird, unter 18 dB (µV/M) reduziert.

14. Handstück (104, 204, 604) nach Anspruch 1, wobei der Schirm gegen elektromagnetische Störungen (270) die mittlere abgestrahlte Emission (EMI), die durch das zahnärztliche Lasergerät während des Simmerbetriebsmodus erzeugt wird, um einen Faktor des 5 bis 10-fachen im Vergleich zu einem nicht abgeschirmten Betriebsmodus bei einer Frequenz von 100 MHz reduziert.

15. Handstück (104, 204, 604) nach Anspruch 1, wobei der Schirm gegen elektromagnetische Störungen die Größenordnung der EMI-Schwankung über mindestens einen Teil von Betriebsfrequenzen zwischen 30 und 1000 MHz reduziert.

## Revendications

1. Pièce à main dentaire à énergie laser comprenant :
un boîtier enfermant :
au moins un émetteur d'énergie laser (208), comprenant au moins un milieu laser à l'état solide (210) et une source de pompage laser (212) ; et
une enveloppe de blindage contre les interférences électromagnétiques (EMI) (150, 170, 270).

2. Pièce à main (104, 204, 604) selon la revendication 1, dans laquelle l'enveloppe de blindage EMI (150, 170, 270) enveloppe au moins l'émetteur d'énergie laser (208).

3. Pièce à main (104, 204, 604) selon la revendication 1, dans laquelle le milieu laser (210) est au moins un milieu laser sélectionné à partir d'un groupe de milieux laser comprenant des lasers Erbium.

4. Pièce à main (104, 204, 604) selon la revendication 1, dans laquelle la source de pompage laser (212) est au moins une source de pompage laser sélectionnée à partir d'un groupe de sources de pompage laser comprenant une lampe à éclairs et un réseau de diodes laser.

5. Pièce à main (104, 204, 604) selon la revendication 1, dans laquelle le blindage contre les interférences électromagnétiques comprend au moins une couche conductrice électrique (274) et au moins deux couches d'isolation électrique (276-1 et 276-2) et dans lequel les deux couches d'isolation électrique (276-1 et 276-2) comprennent au moins un matériau d'isolation électrique sélectionné à partir d'un groupe de matériaux isolants comprenant des polymides, le PVC et des polymères composites.

6. Pièce à main (104, 204, 604) selon la revendication 1, dans laquelle le blindage contre les interférences électromagnétiques (270) comprend au moins une parmi une couche de cuivre et une couche de polymide, toutes deux prises en sandwich par au moins deux couches en polymère.

7. Pièce à main (104, 204, 604) selon la revendication 6, dans laquelle le blindage contre les interférences électromagnétiques (270) est mis à la masse par le biais d'au moins un couplage électriquement conducteur (312) couplé à une extrémité de la couche conductrice électrique (274) et à l'autre extrémité à une masse.

8. Pièce à main (104, 204, 604) selon la revendication 1, dans laquelle la pièce à main (104, 204, 604) comprend également au moins un conduit de fluide (308, 310) communiquant avec une source de fluide (124) ou un réservoir de dépôt de déchets.

9. Pièce à main (104, 204, 604) selon la revendication 1, dans laquelle la pièce à main est couplée par le biais d'un faisceau (206, 606) à une source d'alimentation électrique, une masse, une source de fluide (124) et un réservoir de dépôt de déchets et dans lequel le harnais (206, 606) est multi-luminal.

10. Pièce à main (104, 204, 604) selon la revendication 9, dans laquelle la pièce à main est couplée de manière conductrice à la masse et à la source d'alimentation électrique (108) par le biais de conducteurs électriques logés dans le faisceau et sont également enfermés dans un blindage contre les interférences électromagnétiques.

11. Pièce à main (104, 204, 604) selon la revendication 1, dans laquelle le blindage contre les interférences électromagnétiques (270) réduit l'émission électromagnétique rayonnée moyenne durant un mode de fonctionnement d'effet laser au-dessous de 18 dB (µV/M).

12. Pièce à main (104, 204, 604) selon la revendication 1, dans laquelle le blindage contre les interférences électromagnétiques (270) réduit l'émission rayonnée moyenne (EMI) générée par l'équipement dentaire laser durant un mode de fonctionnement d'effet laser d'un facteur de 5 à 10 dB à une fréquence de 100 MHz.

13. Pièce à main (104, 204, 604) selon la revendication 1, dans laquelle le blindage contre les interférences électromagnétiques (270) réduit l'émission rayonnée moyenne (EMI) générée par le dispositif dentaire laser durant un mode de fonctionnement d'entretien au-dessous de 18 dB(µV/M).

14. Pièce à main (104, 204, 604) selon la revendication 1, dans laquelle le blindage contre les interférences électromagnétiques (270) réduit l'émission rayonnée moyenne (EMI) générée par le dispositif dentaire laser durant un mode de fonctionnement d'entretien d'un facteur de 5 à 10 fois par rapport à un mode de fonctionnement non blindé à une fréquence de 100 MHz.

15. Pièce à main (104, 204, 604) selon la revendication 1, dans laquelle le blindage contre les interférences électromagnétiques réduit l'amplitude de la fluctuation EMI sur au moins une portion de fréquences de fonctionnement entre 30 et 1000 MHz.
